**Europäisches Patentamt**

**European Patent Office**

**Office européen des brevets**

(19)

(11) Veröffentlichungsnummer: **0 238 032**
**A2**

(12) **EUROPÄISCHE PATENTANMELDUNG**

(21) Anmeldenummer: **87103820.4**

(22) Anmeldetag: **17.03.87**

(51) Int. Cl.⁴: **A61F 9/02 , A41D 13/00**

(30) Priorität: **17.03.86 DE 3608872**
**13.03.87 DE 3708052**

(43) Veröffentlichungstag der Anmeldung:
**23.09.87 Patentblatt 87/39**

(84) Benannte Vertragsstaaten:
**AT BE CH DE ES FR GB GR IT LI LU NL SE**

(71) Anmelder: **Schröder, Michael**
**Konviktstrasse 10 a**
**D-7800 Freiburg i. Br.(DE)**

(72) Erfinder: **Schröder, Michael**
**Konviktstrasse 10 a**
**D-7800 Freiburg i. Br.(DE)**

(54) **Pollenschutzvorrichtung.**

(57) Die in Brillenform ausgebildete Pollenschutzvorrichtung besteht aus einem rahmenartigen
Gestell (2), das in seiner Gebrauchslage Augen und
Nase des Benutzers pollendicht umschließt und mittels eines Haltebandes (3) am Kopf gehalten wird.
Einzelne oder sämtliche Wandabschnitte (10,11,14,
15,16) des im wesentlichen U-förmig profilierten Gestells (2) sind als Luftfilter ausgebildet. Als bevorzugtes Filtermaterial wird erfindungsgemäß ein
Polypropylen-Endlosfilament mit einer Rückhalterate
von 2 μm bei Gasfiltration vorgeschlagen. Die mit
solchem Filtermaterial ausgerüstete Schutzbrille (1)
vermittelt einen wirksamen Schutz vor krankheitsübertragenden Bakterien und somit vor
Tröpfcheninfektionen ebenso wie vor anderen
gesundheitsschädlichen Schmutzpartikeln vergleichbar geringer Größe, beispielsweise Asbeststäuben,
die an bestimmten Arbeitsplätzen anfallen können.

FIG. 2

## Pollenschutzvorrichtung

Die Erfindung betrifft eine Pollenschutzvorrichtung, die die Schleimhäute der Augen und des Nasenrachenraumes vor dem Kontakt mit Blütenpollen und anderen gesundheitsgefährdenden Partikeln, beispielsweise krankheitsübertragenden Bakterien, schützen soll.

Unter den Allergosen hat vor allem der Heuschnupfen eine weite Verbreitung gefunden. Ausgelöst wird diese allergische Krankheit alljährlich beim Blühen bestimmter Gräser, Kräuter, Sträucher und Bäume durch deren Blütenpollen, die durch Wind und thermische Strömungen in die Atmosphäre getragen werden. Diese Pollen haben in ihrer Mehrzahl einen Durchmesser von etwa 15 bis 40 μm.

Darüber hinaus besteht aber auch das Bedürfnis, sich vor krankheitsübertragenden Bakterien zu schützen. Von den betroffenen Patienten werden die an Tröpfchen, insbesondere an Speicheltröpfchen gebundenen Bakterien als Aerosol ausgeatmet. Der von mehreren Parametern abhängige Partikeldurchmesser dieses Aerosols beträgt etwa 2 bis 10 μm.

Als Primärschutz der diesbezüglich empfindlichen Personen vor Pollen sind bisher in bezug auf eine mechanische und allergisierte Wirkungsweise prinzipiell zwei Maßnahmen bekanntgeworden.

Zum einen ist eine Pollenschutzhaube bekannt, die aus einem Filtergewebe mit eingearbeitetem Sichtfenster in Verbindung mit einer Kopfbedeckung besteht. Eine solche Haube läßt sich weder unauffällig in der Öffentlichkeit tragen noch in jedem Falle mit leichter Sommerkleidung in harmonische Übereinstimmung bringen. Überdies ist der gesamte Kopf des Betroffenen maskiert, was insbesondere bei hoher Außentemperatur zu erhöhter Schweißbildung führt.

Zum andern sind insbesondere aus Schaumstoff bestehende, konisch geformte Pfropfen bekannt, die in die Nasenkanäle einzuführen sind. Diese Stöpsel werden wegen ihrer Druckausübung auf die Nasenwände als unangenehme Fremdkörper empfunden und aus hygienischen Gründen als keinesfalls unbedenklich angesehen. Außerdem vermitteln sie lediglich den Nasenschleimhäuten einen Schutz, nicht jedoch den Augen, welche durch Blütenstaubreizung ebenfalls stark in Mitleidenschaft gezogen werden können.

Der Erfindung liegt die Aufgabe zugrunde, eine Vorrichtung zu schaffen, die sowohl die Augen als auch die Nase von Pollenallergikern vor Blütenstaubkontakt wirksam zu schützen vermag. Außerdem sollte die Vorrichtung in der Lage sein zu verhindern, daß Pollen durch den Mund in den Rachenraum gelangen können, und zwar dann, wenn wegen verstopfter Nase das Atmen ausschließlich durch den Mund erfolgen muß. Weiterhin sollte die Vorrichtung das Einatmen krankheitsübertragender Bakterien und anderer gesundheitsgefährdender Partikel vergleichbar kleiner Größe unterbinden. Überdies sollte die Möglichkeit geschaffen werden, ein und dieselbe Vorrichtung wahlweise mit verschiedenen Filtern aus jeweils unterschiedlichen Materialien zu benutzen. Ferner sollte die Vorrichtung leicht zu handhaben sowie ohne gesundheitliche Nachteile benutzbar sein. Schließlich sollte die Vorrichtung auch ästhetischen Gesichtspunkten gerecht werden.

Diese Aufgabe wird erfindungsgemäß durch die Merkmale des Hauptanspruchs und der Unteransprüche gelöst.

Die als Brille ausgebildete Pollenschutzvorrichtung gemäß der Erfindung besitzt den Vorteil, daß sie höchstens in zwei Größen -eine für Kinder, die andere für Erwachsene -hergestellt und auf dem Markt angeboten zu werden braucht, daß sie sich gleich einer Sonnenbrille in jede beliebige Art der Bekleidung, sei es für Beruf, Freizeit oder Sport, ohne zusätzliche Maßnahmen integrieren, bequem handhaben und bei Nichtbenutzung ohne größere Platzbeanspruchung gut unterbringen läßt. Außerdem ist es möglich, die Brille durch entsprechende Farbgebung auf die Kleidung abzustimmen.

Das erfindungsgemäß in die Schenkelwände und/oder in die seitlichen Stegwandabschnitte der Brille jeweils eingelassene Luftfilter kann beispielsweise aus einer galvanisierten Metallfolie mit einer Maschenweite kleiner als 40 μm bestehen. Ein solches Filter ist in der Lage, die Blütenpollen zurückzuhalten und die Atemluft passieren zu lassen. In der Praxis hat sich gezeigt, daß beim Tragen dieser Brille hinreichend genug Atemluft zur Verfügung steht, wenn die gesamte Luftdurchlaßfläche mindestens 4 cm² beträgt. Selbst bei mehrstündigem Tragen der Brille tritt keine Sauerstoffarmut auf.

Ein wirkungsvoller Schutz vor dem Kontakt mit Bakterien läßt sich erfindungsgemäß dadurch herbeiführen, daß als Filtermaterial eine vorzugsweise auf Kunststoffasern basierende Filtermatrix verwendet wird, die bei Gasfiltration eine Rückhalterate von 2 μm aufweist. Als besonders geeignet hat sich ein aus Polypropylen-Endlosfilament bestehendes Filtermaterial erwiesen. Dieses hochelastische und zugleich stabile Filter besitzt eine absolute, das heißt in Flüssigkeit validierte Rückhalterate von 20 μm. Wird dieses Filter als Gasfilter eingesetzt, wie dies beim Filtern bakterienbeladener Aerosole der Fall ist, so besitzt es eine zehnfach bessere Abscheideleistung, das heißt die Rückhalterate beträgt 2 μm. Dieses Filter fängt die in Speicheltröpfchen lebenden Bakterien ab und schützt den Träger einer mit diesem Filter ausgerüsteten Brille vor einer Tröpfcheninfektion.

Dieses Polypropylen-Filter gewährleistet, daß einerseits selbst bei hohen Drücken keine filtrierten Teilchen durchbrechen und andererseits keine Faserstücke freigesetzt werden, die in die Lunge gelangen und eine Fibrose hervorrufen könnten. Außerdem zeichnet sich dieses Filter trotz seiner Engmaschigkeit durch einen sehr geringen Atemwiderstand aus.

Dieses Filter wird durch eine Schmelzverschweißung seines Filamentes hergestellt und enthält deshalb keine Kleber oder Harze, die sich in gelöster Form gesundheitsschädigend auswirken könnten. Darüber hinaus ist dieses Filter chemisch inert und kann deshalb bedenkenlos mit alkoholischen Lösungen in Berührung kommen, die eventuell zum Reinigen der Sichtscheibe benutzt werden.

Die mit einem solchen Filter ausgerüstete Brille vermag ihren Benutzer vor Luftverunreinigungen jeder Art zu schützen. So vermittelt sie auch am luftverschmutzten Arbeitsplatz einen wirksamen Schutz, beispielsweise vor den äußerst gesundheitsschädlichen Asbeststäuben.

Die Möglichkeit, mit ein und derselben Brille Filter unterschiedlichen Materials oder verschiedener Maschenweite zu benutzen, ist erfindungsgemäß dadurch herbeigeführt, daß das Filtermaterial auf kassettenförmige Rähmchen aufgespannt ist, die sich als Auswechselteile in entsprechend ausgebildete Halterungen der Wandabschnitte der Brille einsetzen lassen.

Eine gute Be-und Entlüftung des von der Brille umspannten Raumes ist erfindungsgemäß insbesondere dann erreicht, wenn sowohl die untere als auch die obere Schenkelwand jeweils mit einem Luftfilter ausgerüstet ist. In diesem Falle verlaufen nämlich die Luftfilterflächen im wesentlichen parallel zueinander, so daß die Durchtrittsöffnungen der beiden Filter in derselben Strömungsrichtung liegen. Außerdem bewirkt die Anordnung eines Luftfilters in der oberen Schenkelwand einen raschen Abzug der ausgeatmeten Luft, die aufgrund ihres Durchganges durch den menschlichen Körper im allgemeinen wärmer als die Außenluft ist und infolgedessen innerhalb des von Brille und Gesicht gebildeten Raumes nach oben zur oberen Schenkelwand hin steigt und durch das dortige Filter sofort ins Freie entweichen kann. Dem Kondensieren von Wassertröpfchen an der Sichtscheibe kann zusätzlich noch dadurch entgegengewirkt werden, daß als Material für die Scheibe ein sogenanntes Antibeschlagglas verwendet wird.

Ein weiteres Kennzeichen der erfindungsgemäßen Schutzbrille besteht darin, daß in die untere Schenkelwand ein die Ausatemluft hindurchlassendes Ventil, vorzugsweise eine Gummimembran, eingelassen ist. Dieses Ventil, das durch den beim Ausatmen freigesetzten Luftstrom in Öffnungsstellung gebracht wird, erlaubt ein rasches Austreten der Atemluft ins Freie. Außerdem ermöglicht das geöffnete Ventil das Passieren kleinster Wasserpartikel, die unter Umständen in dem von der Brille umschlossenen Raum entstehen und von der ausgeatmeten Luft mitgerissen werden.

Zur Kanalisierung der Ausatemluft dienen in vorteilhafter Weise erfindungsgemäß zwei von der Innenseite der unteren Schenkelwand ausgehende, quer zu deren Längsrichtung in gegenseitigem Abstand angeordnete Luftleitflügel, die bei Benutzung der Brille die Nase des Benutzers flankieren.

Des weiteren ist die Erfindung dadurch gekennzeichnet, daß die untere Schenkelwand eine in die Unterseite ihres an der offenen Gestellseite liegenden Randteils eingelassene hinterschnittene Nut aufweist, mit welcher ein in seinem Querschnitt dem Nutquerschnitt angepaßtes Verbindungsteil wie Rundstab formschlüssig verbindbar ist, welches seinerseits als Träger für ein bis zum Hals des Benutzers reichendes Schutztuch dient, das an seiner unteren Längskante als Hohlsaum zur Aufnahme einer Befestigungsschnur ausgebildet ist. Dieses Schutztuch wird mit Vorteil dann zu Hilfe genommen, wenn wegen verstopfter Nase das Atmen nur durch den Mund möglich ist. Das mit der vorgenannten Nut in Eingriff gebrachte Tuch wird mit seinem unteren Teil um den Hals des Brillenbenutzers gelegt und mittels der durch den Hohlsaum laufenden Schnur festgezogen, so daß es randseitig rundum dicht am Gesicht anliegt. Dieses Schutztuch besteht aus einem atmungsaktiven, pollenundurchlässigen Stoff.

Ein zuverlässiger, pollendichter Sitz der mittels eines vorzugsweise elastischen Haltebandes am Kopf des Benutzers gehaltenen Schutzbrille ist nicht zuletzt erfindungsgemäß dadurch erreicht, daß der freie Längsrand jeder Schenkel wand mit einem elastisch nachgiebigen, pollenundurchlässigen Dichtungsstreifen abgepolstert ist.

In vorteilhafter Weise besteht das Gestell der Schutzbrille erfindungsgemäß aus biegsamem Material wie Polyäthylen, so daß es weder leicht zerbrechen kann noch eine Verletzungsgefahr für den Benutzer darstellt.

Die Erfindung wird nachfolgend anhand von zwei in der Zeichnung in verkleinertem Maßstab dargestellten Ausführungsbeispielen des näheren erläutert. In der Zeichnung zeigen, jeweils in perspektivischer Darstellung:

Fig. 1 eine auf den Kopf eines Benutzers aufgesetzte Schutzbrille gemäß der Erfindung,

Fig. 2 dieselbe Schutzbrille allein,

Fig. 3 einen vergrößerten Ausschnitt einer abgewandelten Ausführung einer Schutzbrille,

Fig. 4 die Ansicht der Schutzbrille gemäß Fig. 2 von unten und

Fig. 5 einen vergrößerten Teilabschnitt der unteren Schenkelwand gemäß Fig. 4 mit eingehängtem Schutztuch.

Die im ganzen mit 1 bezeichnete Schutzbrille gemäß der Erfindung besteht aus einem rahmenartigen Gestell 2 aus biegsamem Material wie Polyäthylen und einem an diesem angelenkten Halteband 3 aus vorzugsweise gummi-elastischem Material. Das Gestell 2 ist im wesentlichen U-förmig profiliert und hat einen mondsichelförmigen Grundriß. Die beiden Schenkelwände 4 und 5 des Gestells 2 sind zwecks Anpassung an die Gesichtskonturen des Brillenbenutzers an ihren freien Längsrändern konkav gekrümmt und zur Erzielung eines guten wie ermüdungsfreien Sitzes mit je einem elastisch nachgiebigen, pollenundurchlässigen Dichtungsstreifen 6 bzw. 7 abgepolstert.

Die Stegwand 8 des Gestells 2 besteht zum größten Teil aus einer einen Panoramablick gewährenden Sichtscheibe 9 aus unzerbrechlichem Material, beispielsweise Polykarbonat. Die beiden seitlichen Stegwandabschnitte 10 und 11 sind als Luftfilterelemente ausgebildet. Auch in den beiden Schenkelwänden 4 und 5 sind je nach Bedarf in einzelnen oder sämtlichen Wandabschnitten 14, 15, 16 und 17 Luftfilterelemente untergebracht.

In der Mitte der unteren Schenkelwand 5 ist ein bei Druckbeaufschlagung durch die nasal ausgeatmete Luft sich öffnendes Ventil in Form einer Gummimembran 18 untergebracht. Zu beiden Seiten der Gummimembran 18 ist quer zur Längsrichtung der unteren Schenkelwand 5 je ein Luftleitflügel 19 bzw. 20 angeordnet.

Die untere Schenkelwand 5 weist in dem dem Brillenträger zugewandten Randteil 21 an dessen Unterseite eine V-förmige Nut 27 auf, in welche der entsprechend geformte Zapfen 29 der Schiene 28 eingreift, wobei die Schiene 28 mittels eines Klebers mit dem Randteil 21 verbunden ist. In die Schiene 28 ist eine in Längsrichtung sich erstreckende hinterschnittene Nut 22 eingelassen. Das Schutztuch 24 ist mit seinem oberen Längsrand an einem Rundstab 23 befestigt und kann durch Einschieben des Rundstabes 23 in die Nut 22 an der Schiene 28 bzw. an dem Randteil 21 aufgehängt werden. Der untere Längsrand des Schutztuches 24 ist als Hohlsaum 25 ausgebildet, durch welchen eine Befestigungsschnur 26 gezogen ist. Mittels dieser Schnur 26 läßt sich das Schutztuch 24 am Hals des Brillenbenutzers festbinden.

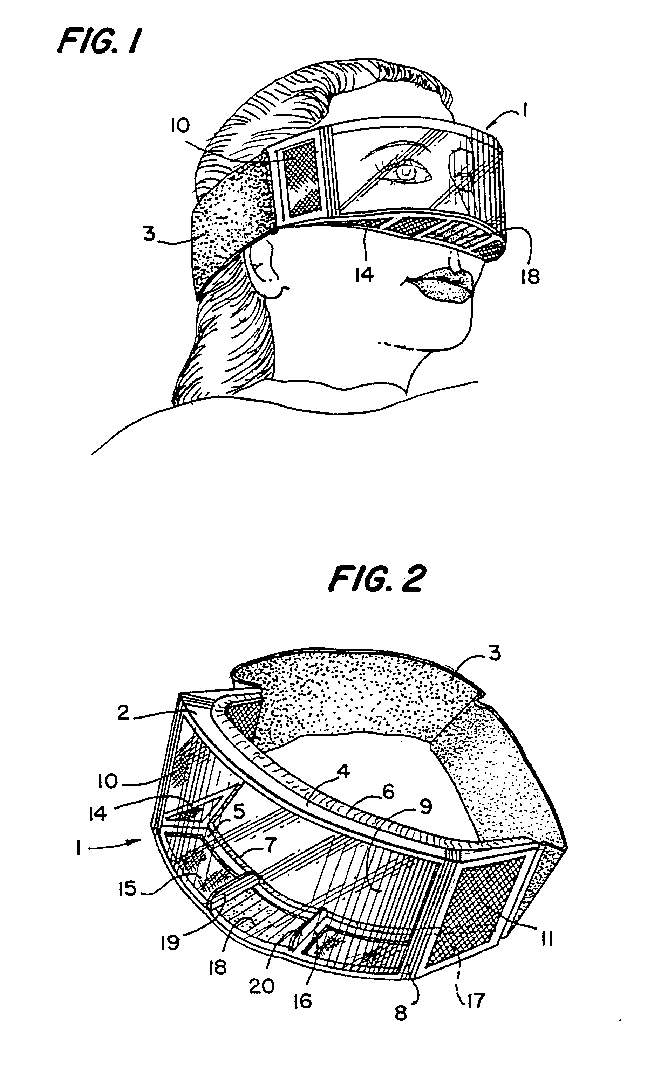

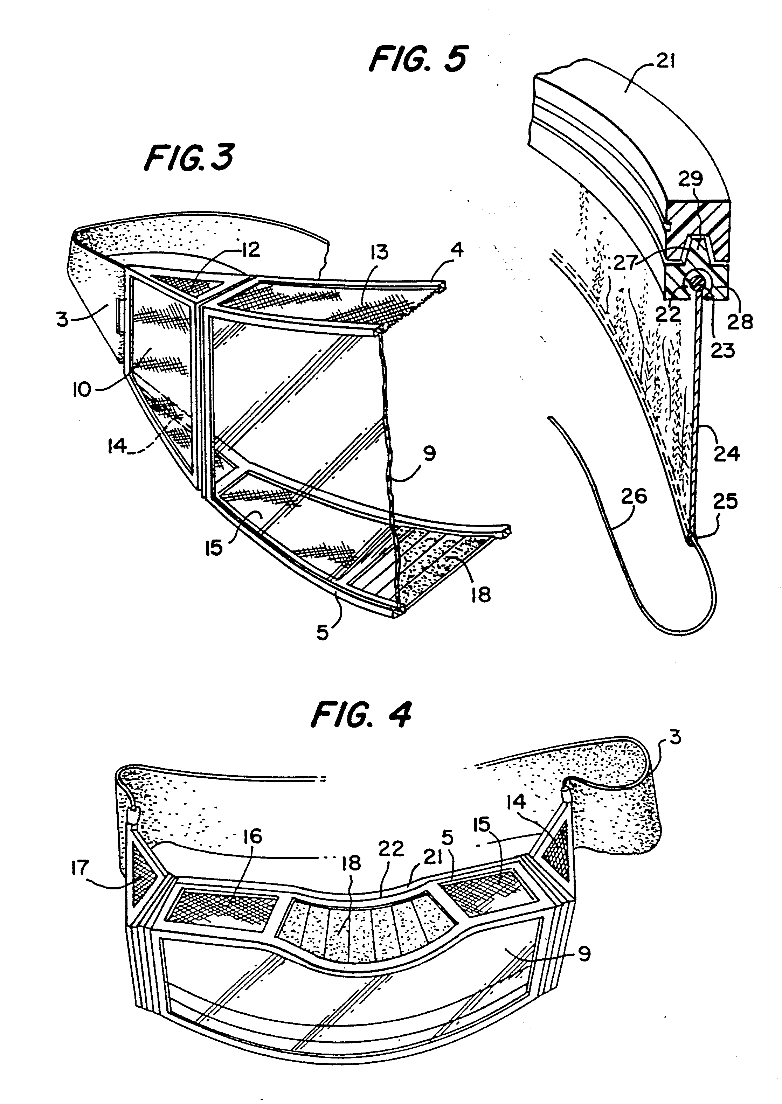

Fig. 3 zeigt eine abgewandelte Ausführung einer Schutzbrille gemäß der Erfindung. Diese Ausführung unterscheidet sich von der Ausführung gemäß Fig. 1, 2 und 4 dadurch, daß auch in der oberen Schenkelwand 4, und zwar in deren Wandabschnitten 12 und 13 sowie in den in derselben Ebene liegenden, jedoch nicht eingezeichneten spiegelbildlichen Wandabschnitten Filterelemente angeordnet sind.

Die zur Unterbringung in den Wandabschnitten 10,11,12,13, 14,15,16 und 17 der gezeigten Brillenausführungen vorgesehenen Filterelemente können aus unterschiedlichem Material sein. Allein zum Filtern von Blütenpollen kann eine galvanisierte Metallfolie mit einer unter 40 μm liegenden Maschenweite benutzt werden. Zum Fernhalten von Bakterien dagegen ist ein Filter aus Polypropylen-Endlosfilament mit einer Rückhalterate von 2 μm bei Gasfiltration gemäß der Erfindung als besonders geeignet anzusehen. Das Filtermaterial kann auf -in der Zeichnung nicht dargestellte -kassettenförmige Rähmchen aufgespannt sein, die sich als Auswechselteile in entsprechend ausgebildete, ebenfalls nicht dargestellte Halterungen der Wandabschnitte 10,11,12,13,14,15,16 und 17 der Schutzbrille 1 einsetzen lassen.

**Ansprüche**

1. Pollenschutzvorrichtung, **gekennzeichnet durch** die Form einer Brille, bestehend aus einem rahmenartigen Gestell (2), das in seiner Gebrauchslage Augen und Nase des Brillenbenutzers pollendicht umschließt und mittels eines elastischen oder auf die gewünschte Länge einstellbaren Haltebandes - (3) am Kopf gehalten wird, wobei die Stegwand (8) des im wesentlichen U-förmig profilierten Gestells - (2) aus einer einen Panoramablick gewährenden Sichtscheibe (9) besteht sowie mindestens eine der beiden zur Sichtscheibe (9) im wesentlichen senkrecht verlaufenden Schenkelwände (4, 5) und/oder mindestens einer der an die Sichtscheibe (9) seitlich sich anschließenden Stegwandabschnitte - (10,11) teilweise oder ganz als Luftfilter, vorzugsweise in Form einer galvanisierten Metallfolie, mit einer Maschenweite kleiner als 40 μm ausgebildet ist.

2. Pollenschutzvorrichtung nach Anspruch 1, **dadurch gekennzeichnet,** daß als Filtermaterial eine vorzugsweise auf Kunststofffasern basierende Filtermatrix mit einer Rückhalterate von 2 μm bei Gasfiltration verwendet wird.

3. Pollenschutzvorrichtung nach Anspruch 2,
**dadurch gekennzeichnet,**
daß des Filtermaterial aus einem Polypropylen-Endlosfilament besteht.

4. Pollenschutzvorrichtung nach Anspruch 1,
**dadurch gekennzeichnet,**
daß das Luftfilter auswechselbar angeordnet ist.

5. Pollenschutzvorrichtung nach einem der Ansprüche 1-4,
**dadurch gekennzeichnet,**
daß das Filtermaterial auf kassettenförmige Rähmchen aufgespannt ist.

6. Pollenschutzvorrichtung nach Anspruch 5,
**dadurch gekennzeichnet,**
daß die Wandabschnitte (14,15,16,17) der Brille (1) mit Halterungen zur Aufnahme der kassettenförmigen Rähmchen versehen sind.

7. Pollenschutzvorrichtung nach Anspruch 1,
**dadurch gekennzeichnet,**
daß jede Schenkelwand (4,5) an ihrem freien Längsrand (6,7) konkav gekrümmt ist.

8. Pollenschutzvorrichtung nach Anspruch 1,
**dadurch gekennzeichnet,**
daß die beiden Schenkelwände (6,7) einen mondsichelförmigen Grundriß besitzen.

9. Pollenschutzvorrichtung nach Anspruch 1,
**dadurch gekennzeichnet,**
daß der freie Längsrand (6,7) jeder Schenkelwand (4,5) mit einem elastisch nachgiebigen, pollenundurchlässigen Dichtungsstreifen abgepolstert ist.

10. Pollenschutzvorrichtung nach Anspruch 1,
**dadurch gekennzeichnet,**
daß in die untere Schenkelwand (5) ein die Ausatemluft hindurchlassendes Ventil eingelassen ist.

11. Pollenschutzvorrichtung nach Anspruch 10,
**dadurch gekennzeichnet,**
daß das Ventil aus einer Gummimembran (18) besteht.

12. Pollenschutzvorrichtung nach Anspruch 1,
**gekennzeichnet durch**
zwei von der Innenseite der unteren Schenkelwand (5) ausgehende, quer zu deren Längsrichtung in gegenseitigem Abstand angeordnete Luftleitflügel - (19,20), die bei Benutzung der Vorrichtung die Nase des Benutzers flankieren.

13. Pollenschutzvorrichtung nach Anspruch 1,
**dadurch gekennzeichnet,**
daß die untere Schenkelwand (5) eine in die Unterseite ihres an der offenen Gestellseite liegenden Randteils (21) eingelassene hinterschnittene Nut - (22) aufweist, mit welcher ein in seinem Querschnitt dem Nutquerschnitt angepaßtes Verbindungsteil wie Rundstab (23) formschlüssig verbindbar ist, welches seinerseits als Träger für ein bis zum Hals des Benutzers reichendes Schutztuch - (24) dient, das an seiner unteren Längskante als Hohlsaum (25) zur Aufnahme einer Befestigungsschnur (26) ausgebildet ist.

14. Pollenschutzvorrichtung nach Anspruch 1,
**dadurch gekennzeichnet,**
daß das Gestell (2) aus biegsamem Material wie Polyäthylen besteht.

## FIG. 1

## FIG. 2

*FIG. 5*

*FIG.3*

*FIG. 4*